# EUROPEAN PATENT APPLICATION

(11) **EP 1 717 221 A1**
(43) Date of publication of application: **02.11.2006**
(21) Application number: 05710165.1
(22) Date of filing: 14.02.2005
(51) Int. Cl.: C07C 239/20, C07C 45/61, C07C 49/497, C07C 49/17, C07C 47/19, C07C 47/27, B01J 31/02, C07D 211/74, C07D 309/30, C07D 317/72, C07B 53/00, C07D 403/04

(54) **PROCESSES FOR PRODUCTION OF ALPHA-AMINOOXYKETONES AND ALPHA-HYDROXYKETONES**

(30) Priority: 20.02.2004 JP 2004044540
(71) Applicant: Japan Science and Technology Agency, Kawaguchi-shi, Saitama 332-0012 (JP)
(72) Inventor: SAITO, Susumu, Nagoya-shi, Aichi 4640077 (JP)
(74) Representative: Alcock, David
(86) International application number: PCT/JP2005/002163
(87) International publication number: WO 2005/080318

(57) **Abstract**

The present invention is to provide a method for easily obtaining α-aminooxyketone compound which is a synthetic equivalent for monosaccharide and pentoses, and a equivalent of α-hydroxyketone compound that can be synthetic intermediates of various physiologically active materials, in high yield; to pave the way for the synthesis of monosaccharide and furthermore of oligosaccharide from the resulting α-hydroxyketone compound induced from α-aminooxyketone compound; and to open new possibilities for the synthesis of various sugar medicines such as anticancer agents, antithrombogenic agents, anti-HIV agents, inhibitors of cholesterol synthesis, verotoxin neutralizing agents. According to the invention, a carbonyl compound is allowed to react with a nitroso compound to produce an α-aminooxyketone compound using a catalyst containing a heterocyclic compound shown in the general formula (I) (wherein X1, X2 and X3 independently represent nitrogen, carbon, oxygen or sulfur; and Z represents a substituted or unsubstituted 5-to 10-membered ring).

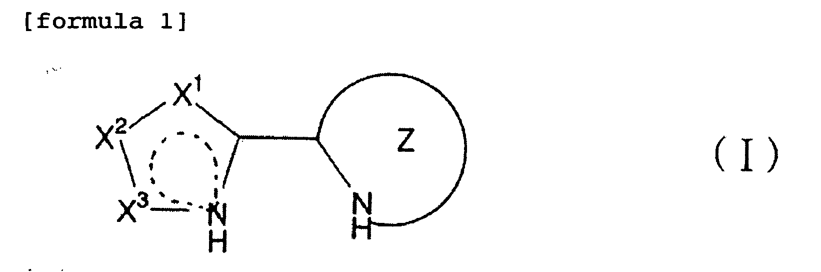

## Description

### Technical Field

The present invention relates to a medicine, an organic material having physiological activity, α-aminooxyketone compounds which are materials of liquid crystal and the like, and a process for producing α-hydroxyketone compounds induced therefrom; as to a catalyst used for producing α-aminooxyketone compounds, specifically α-aminooxyketone compounds which can give optical isomers with high purity, and a process for producing α-hydroxyketone compounds induced therefrom, and a catalyst used for producing α-aminooxyketone compounds.

### Background Art

α-hydroxycarbonyl compounds are frequently found in natural organic compounds and the molecule skeletons of pharmaceuticals. They are synthetic equivalents for monosaccharides and pentoses, and are very important synthetic building blocks which can be led to various physiologically active materials, intermediates in the synthesis of medicines and liquid crystalline materials. The α-hydroxycarbonyl compounds easily lead to synthesis of optical isomers with high purity by asymmetric oxygenation of carbonyl compounds. However, asymmetric oxygenation at the α-position of the carbonyl group by the usual methods requires a two-step process: first, synthesizing and isolating of enolate intermediates from carbonyl compounds, and second, performing with the use of a relatively expensive oxygen-introducing reagent; therefore, the asymmetric oxygenation has problems of low atom efficiency and the like.

In contrast, as a method for asymmetric oxygenation of ketones, methods for directly obtaining asymmetric oxygenation products of ketones without synthesizing and isolating of enolate intermediates have been reported. The asymmetric oxygenation is to obtain α-aminooxyketones using amino acid, proline as a catalyst and nitrosobenzene as an oxygen-introducing reagent (see e.g. non-patent documents 1-3). However, many problems have so far not been elucidated in these systems, including low catalytic efficiency (10 to 20 mol % catalyst are needed), poor reproducibility and the like. Moreover, it is known that double oxygenation by nitrosobenzene progresses a side reaction.

Alternatively, it has been reported that α-aminooxyketone can be obtained in high yield from an alkylsilyl enol ether and nitrosobenzene with alkylsilyl triflate as a Lewis Acid catalyst (see e.g. Nonpatent document 4) and also from an alkyltin enolate and nitrosobenzene with Ag-BINAP complex as a catalyst (see e.g. Nonpatent document 5). Additionally, other methods have been disclosed to produce aldol products from the condensation reaction of carbonyl compounds: by using a compound which has an ether and alcohol units within a certain molecule, in liquid CO₂ or supercritical CO₂, in the presence of an acid catalyst (see e.g. Patent document 1); by performing the reaction in water, in the presence of boron acid, a surfactant or a Brönsted acid (see e.g. Patent document 2); by using a lanthanide trifluoromethanesulfonate and a chiral crown ether as a catalyst (see e.g. Patent document 3); and the like.
Patent document 1: Japanese Laid-Open Patent Application No. 2002-284729
Patent document 2: Japanese Laid-Open Patent Application No. 2002-275120
Patent document 3: Japanese Laid-Open Patent Application No. 2002-200428
Nonpatent document 1: Brown, S. P., Brochu, M. P., Sinz, C. J., & MacMillan, D. W. C. (2003) J. Am. Chem. Soc. 125, 10808-10809
Nonpatent document 2: Zhong, G. (2003) Angew. Chem. Int. Ed. 42, 4247-4250
Nonpatent document 3: Hayashi, Y., Yamaguchi, J., Hibino, K., & Shoji, M. (2003) T etrahedron Lett. 44, 8293-8296
Nonpatent document 4: Momiyama, N., Yamamoto, H. (2002) Angew. Chem. Int. Ed. 41, 2986-2987
Nonpatent document 5: Momiyama, N., Yamamoto, H. (2003) J. Am. Chem. Soc. 125, 60 38-6039

### Disclosure of the Invention

### Object to be solved by the present invention

Object of the present invention is to provide a method for easily obtaining α-aminooxyketone compound which is a synthetic equivalent for monosaccharide and pentoses, and a equivalent of α-hydroxyketone compound that can be a synthetic intermediates of various physiologically active materials, in high yield; to pave the way for the synthesis of monosaccharide and furthermore of oligosaccharide from the resulting α-hydroxyketone compound induced from α-aminooxyketone compound; and to open new possibilities for the synthesis of various sugar medicines such as anticancer agents, antithrombogenic agents, anti-HIV agents, inhibitors of cholesterol synthesis, verotoxin neutralizing agents.

### Means to solve the object

The present inventor, as shown in the following formula, in trying on an asymmetric nitroso aldol reaction between 3.0 equivalents of cyclohexanone (2a) and 1.0 equivalent of nitrosobenzene (3) using pyrrolidine tetrazole type catalyst (1) (5 mol%) induced from proline, in dimethyl sulfoxide (DMSO) at room temperature 25-30 °C, the reaction was almost completed in only one hour, and the desired product (4a) was obtained in 94% chemical yield and >99%ee optical purity.

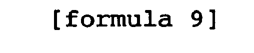

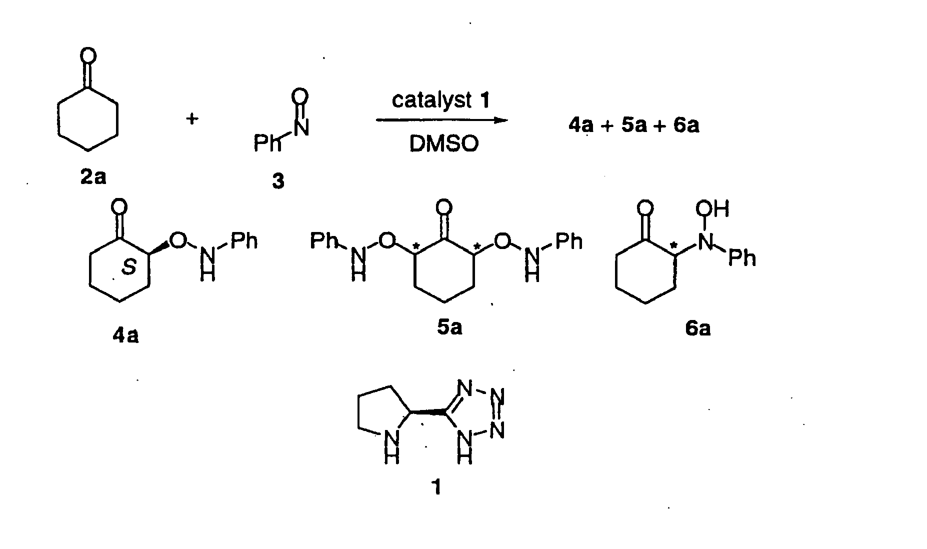

Under the exactly equal reaction condition, when proline was used as a catalyst, chemical yield stayed at 35% and at the same time almost 2% of the production of double additional product (5a) was identified. Even when the amount of catalyst to be used (1) was converted from 5 mol% to 3 mol%, and to 2 mol%, the reactions were progressed to observe a little decrease of the chemical yield to 72% and 50% respectively, but the optical purities were very high with >99%ee. Further, various carbonyl compounds were subjected to reaction, although reaction conditions should be adjusted slightly for aldehydes, high optical purities were obtained in any of the examples. The present invention has been thus completed based on this knowledge.

That is, the present invention relates to:

a process for producing an α-aminooxyketone compound wherein a carbonyl compound is reacted with a nitroso compound by using a catalyst containing a heterocyclic compound shown in the general formula (I) (wherein: X1, X2 and X3 independently represent nitrogen, carbon, oxygen or sulfur; Z represents a substituted or unsubstituted 5- to 10-membered ring) ("1"); 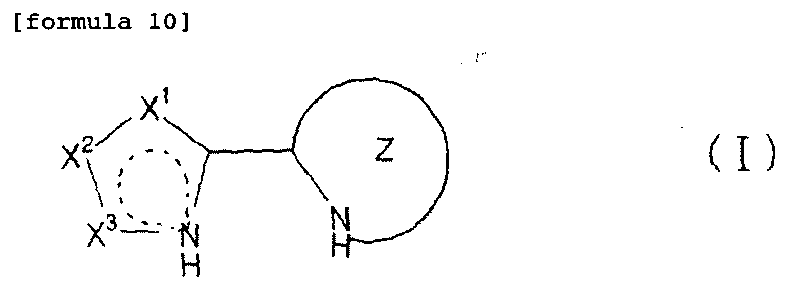 preferably relates to:

the process for producing an α-aminooxyketone compound according to "1", wherein the heterocyclic compound is a tetrazole derivative shown in the general formula (II) (wherein Z represents a substituted or unsubstituted 5- to 10-membered ring) ("2");

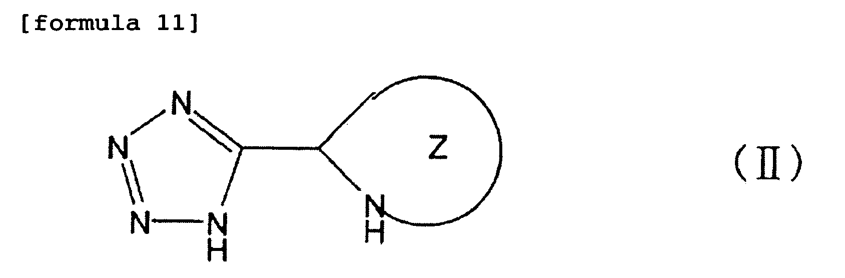

the process for producing an α-aminooxyketone compound according to "2" , wherein the tetrazole derivative is a compound shown in the general formula (III) ("3"); 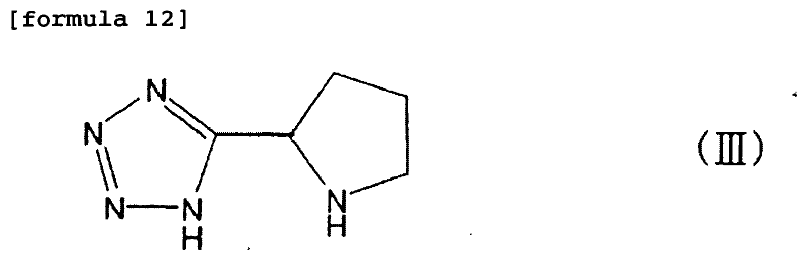 the process for producing an α-aminooxyketone compound according to any one of "1" to "3", wherein the nitroso compound is nitrosobenzene ("4");

the process for producing an α-aminooxyketone compound according to any one of "1" to "4", wherein the carbonyl compound is a compound shown in the general formula (IV) (wherein R1 and R2 independently represent hydrogen, or a substituted or unsubstituted alkyl group, a substituted or unsubstituted alkenyl group, a substituted or unsubstituted alkoxy group, a substituted or unsubstituted amide group, or a substituted or unsubstituted aryl group; and R1 and R2 may bind to each other to form a ring) ("5"); 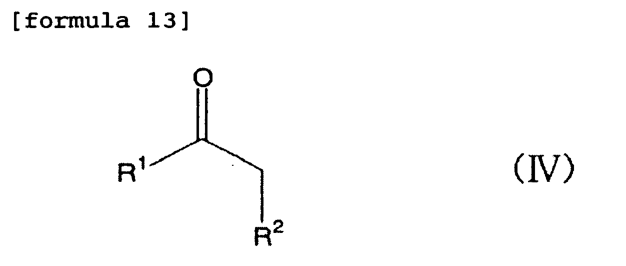 the process for producing an α-aminooxyketone compound according to "5", wherein the α-aminooxyketone compound contains R asymmetric carbon, where R1 represents hydrogen in the general formula (IV) ("6"); the process for producing an α-aminooxyketone compound according to "5", wherein the α-aminooxyketone compound contains S asymmetric carbon; where R1 represents a substituted or unsubstituted alkyl group, a substituted or unsubstituted alkenyl group, a substituted or unsubstituted alkoxy group, a substituted or unsubstituted amide group, or a substituted or unsubstituted aryl group, and either one of these groups bind to R2 to form a ring in the general formula (IV) ("7"); the process for producing an α-aminooxyketone compound according to any one of "1" to "7", wherein a solvent containing dimethyl sulfoxide or methyl nitrile, or both of them is used ("8"); the process for producing an α-aminooxyketone compound according to any one of "1" to "8", wherein reaction is performed at room temperature (20-30 °C) ("9"); and a process for producing an α-hydroxyketone compound wherein an α-aminooxyketone compound obtained from the process according to any one of "1" to "9" is reacted in a solvent by using CuSO₄ as a catalyst ("10").

The present invention also relates to:

a catalyst for producing an α-aminooxyketone compound by which a carbonyl compound is reacted with a nitroso compound, wherein the catalyst contains a heterocyclic compound shown in the general formula (I) (wherein: X1, X2 and X3 independently represent nitrogen, carbon, oxygen or sulfur; Z represents a substituted or unsubstituted 5- to 10-membered ring) ("11"); 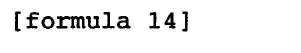 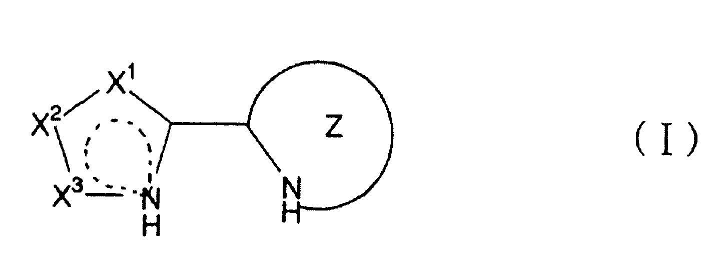 preferably relates to:

the catalyst for producing an α-aminooxyketone compound according to "11", wherein the heterocyclic compound is a tetrazole derivative shown in the general formula (II) (wherein Z represents a substituted or unsubstituted 5- to 10-membered ring) ("12"),

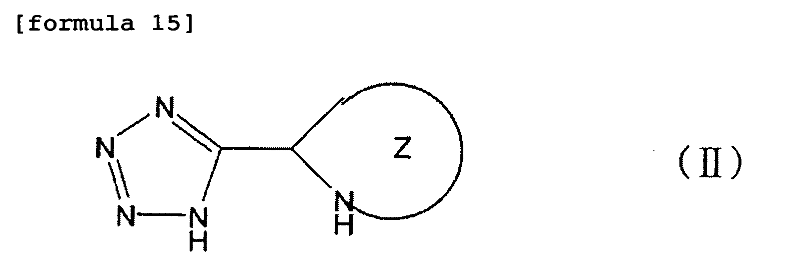

the catalyst for producing an α-aminooxyketone compound according to "12", wherein the tetrazole derivative is a compound shown in the general formula (III) ("13"), 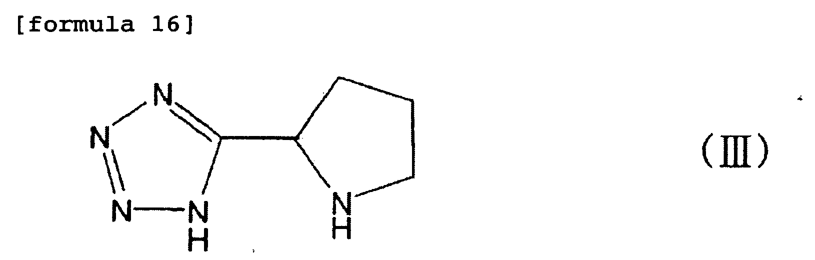 the catalyst for producing an α-aminooxyketone compound according to any one of claims "11" to "13" , wherein the nitroso compound is nitrosobenzene ("14"),

the catalyst for producing an α-aminooxyketone compound according to any one of "11" to "14", wherein the carbonyl compound is a compound shown in the general formula (IV) (wherein R1 and R2 independently represent hydrogen, or a substituted or unsubstituted alkyl group, a substituted or unsubstituted alkenyl group, a substituted or unsubstituted alkoxy group, a substituted or unsubstituted amide group, or a substituted or unsubstituted aryl group; and R1 and R2 may bind to each other to form a ring) ("15"), 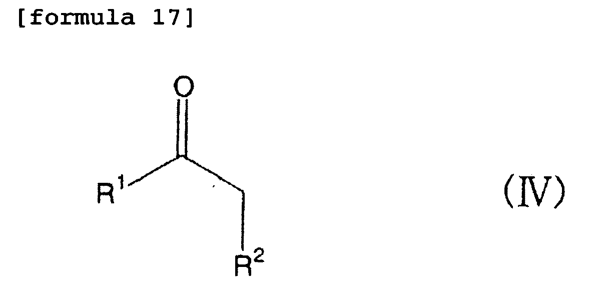 the catalyst for producing an α-aminooxyketone compound according to "15", wherein the α-aminooxyketone compound contains R asymmetric carbon, where R1 represents hydrogen in the general formula (IV) ("16"), the catalyst for producing an α-aminooxyketone compound according to "15", wherein the α-aminooxyketone compound contains S asymmetric carbon, where R1 represents a substituted or unsubstituted alkyl group, a substituted or unsubstituted alkenyl group, a substituted or unsubstituted alkoxy group, a substituted or unsubstituted amide group, or a substituted or unsubstituted aryl group, or where either one of these groups bind to R2 to form a ring in the general formula (IV) ("17").

### Effect of the present invention

By using heterocyclic compound shown in the general formula (I) as a catalyst, without isolating intermediates, at a stage in an asymmetric oxygenation of carbonyl compounds, the amount of catalyst was reduced, the efficiencies of catalyst, atom and the like were increased, and consequently α-aminooxyketone compounds can be obtained in high chemical yield and high optical purity. The present invention paves the way for artificially and freely producing oligosaccharides, hexose and pentose skeletons which can be found in unit structures in DNA and RNA; and gives possibilities to develop various sugar medicines such as anticancer agents, antithrombogenic agents, anti-HIV agents, inhibitors of cholesterol synthesis, and verotoxin neutralizing agents.

### Best Mode of Carrying Out the Invention

A process for producing α-aminooxyketone compounds of the present invention is a process by using so-called nitroso aldol reaction which produces α-aminooxyketone compounds by an asymmetric oxygenation reaction of carbonyl compounds and nitroso compounds.

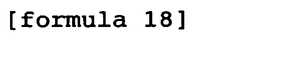

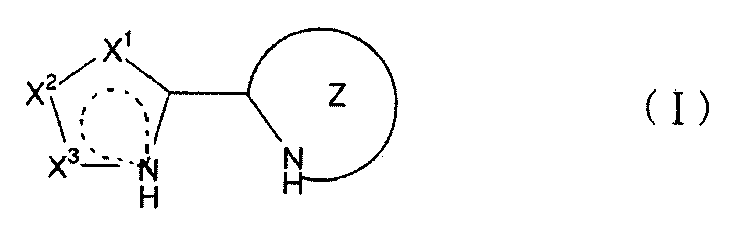

A process for producing α-aminooxyketone compound of the present invention is not especially limited as long as it is a process for producing an α-aminooxyketone compound wherein a carbonyl compound is reacted with a nitroso compound by using a catalyst containing a heterocyclic compound, shown in the general formula (I) (wherein: X1, X2 and X3 independently represent nitrogen, carbon, oxygen or sulfur; Z represents a substituted or unsubstituted 5- to 10-membered ring).

A carbonyl compound used for a process for producing α-aminooxyketone compounds of the present invention is not especially limited as long as it has a carbonyl group, and may be a compound in a soluble form or a compound easily forming hydrates. Specifically, aldehyde compounds and ketone compounds, preferably carbonyl compounds shown in the general formula (IV) can be exemplified.

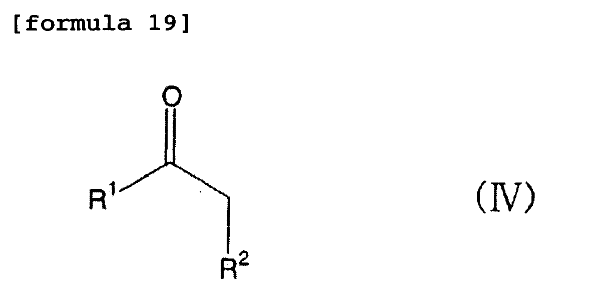

In the general formula (IV), R1 and R2 independently represent hydrogen, or a substituted or unsubstituted alkyl group, a substituted or unsubstituted alkenyl group, a substituted or unsubstituted alkoxy group, a substituted or unsubstituted amide group, or a substituted or unsubstituted aryl group; and R1 and R2 may bind to each other to form a ring. As an alkyl group represented by R1, R2 in the general formula (IV), it may be linear or cyclic, and alkyl groups with 1-30 carbons such as methyl, ethyl, n-propyl, n-butyl, n-pentyl, n-hexyl, n-heptyl, n-octyl, n-nonyl and n-decyl, and such as cyclobutyl, cyclopentyl, cyclohexyl, cycloheptyl, cyclooctyl, cyclononyl and cyclodecyl can be exemplified. As an alkenyl group represented by R1, R2 in the general formula (IV), alkenyl groups with 1-30 carbons such as vinyl, 1-propenyl, allyl, 1-butenyl, 2-butenyl, 1-pentenyl, and 1-hexynyl can be exemplified.

Further, as an alkoxy group, an alkoxycarbonyl group and an aryl group represented by R1, R2 in the general formula (IV), alkoxy groups with 1-30 carbons such as methoxy, ethoxy, n-propoxy, n-butoxy, n-pentyloxy, n-hexyloxy, cyclohexyloxy and phenyloxy; alkoxycarbonyl group with 1-30 carbons such as methoxy carbonyl, ethoxy carbonyl, butoxy carbonyl and pentyloxy carbonyl; and aryl groups with 1-30 carbons such as phenyl, 1-naphtyl, 2-naphthyl, 1-anthryl, 2-anthryl, 9-anthryl, 1-phenanthryl, 2-phenanthryl, benzyl and phenethyl; can be exemplified respectively.

As a substituent of alkyl group, alkenyl group, alkoxy group, alkoxy-carbonyl group and aryl group represented by R1, R2 in the general formula (IV), alkyl groups such as methyl, ethyl, n-propyl, n-butyl, cyclopentyl, cyclohexyl and cycloheptyl; alkenyl groups such as vinyl, 1-propenyl, allyl, and 1-butenyl; aryl groups such as phenyl, 1-naphthyl, 2-naphthyl, 1-anthryl, 2-anthryl, 9-anthryl, 1-phenanthryl, 2-phenanthryl and benzyl; halogen atoms such as F, Cl and Br; alkoxy groups such as methoxy, ethoxy, propoxy and butoxy; and other ones such as hydroxyl, carboxyl, acyl, amino, thio and nitro groups can be exemplified. Further, as a ring system which is formed by binding of R1 and R2, cyclic alkyl groups such as cyclopentane, cyclohexane, cycloheptane, cyclooctane, cyclononane and cyclodecane; aromatic rings such as benzene, naphthalene and anthracene; and heterocycles such as pyridine, pyrrolidine, piperidine, furan, pyran, tetrahydrofuran and tetrahydropyran can be exemplified.

As a carbonyl compound represented in the general formula (IV), specifically aldehydes such as acetaldehyde, propionaldehyde, butylaldehyde, isobutylaldehyde, valeraldehyde, isovaleraldehyde, caproicaldehyde, heptaldehyde, caprylaldehyde, pelargonicaldehyde, capricaldehyde, undecylaldehyde, lauraldehyde, tridecylaldehyde, myristaldehyde, pentadecylaldehyde, palmiticaldehyde, margaricaldehyde, stearicaldehyde, succindialdehyde, acrolein, crotonaldehyde, benzaldehyde, o-tolualdehyde, m-tolualdehyde, p-tolualdehyde, salicylaldehyde, cinnamaldehyde, 1-naphthoaldehyde, 2-naphthoaldehyde and furfural can be exemplified.

Further, as a carbonyl compound represented in the general formula (IV), specifically ketones such as acetone; ethylmethylketone; propylmethylketone; isopropylmethylketone; butylmethylketone; isobutylmethylketone; diethylketone; diisopropylketone; 2-undecanone; methylvinylketone; mesityloxide; fluoroacetone; chloroacetone; 2,4-pentanedione; cyclobutanone; cyclopentanone; cyclohexanone; 2-methylcyclohexanone; cyclodecanone; 2-norbornanone; 2-adamantanone; tetrahydropyrane-4-one; spiro[4,5]-1,4-dioxy-decane-8-one; 1-benzylcarbonylpyperidine-4-one; benzylacetone; 1-indanone; 2-indanone; α-tetralone; β-tetralone; 7- methoxy-2-tetralone; acetophenone; propiophenone; benzylphenone; dibenzylketone; 3,4-dimethylacetophenone; 2-acetonaphthone; and 2-choroloacetophenone can be exemplified.

As a nitroso compound used in a process for producing α-aminooxyketone compounds of the present invention, it may be either of an aliphatic nitroso compound or an aromatic nitroso compound as long as the compound contains a nitroso group. As an aliphatic nitroso compound, an alkyl nitroso compound which may contain substituents can be exemplified and those in which a nitroso group is attached to the tertiary carbon are preferred with specific examples being 2-nitroso-isobutane and 2-nitroso-2-methylpentane. Further, as an aromatic nitroso compound, nitroso benzene which may contain substituents, and 1-nitrosonaphthalene and 2-nitrosonaphthalene which may contain substituents, can be exemplified. As a substituent of aromatic nitroso compound, alkyl groups such as methyl, ethyl, propyl, isopropyl, n-butyl, isobutyl, s-butyl, and t-butyl; alkoxy groups such as methoxy, ethoxy, n-propoxy, isopropoxy, n-butoxy, s-butoxy, isobutoxy, t-butoxy, phenoxy, benzyloxy, and phenethyloxy; and halogen atoms such as F, Cl, Br, and I can be exemplified. As a nitrosobenzene containing substituent group, specifically o-nitrosotoluene; m-nitrosotoluene; p-nitrosotoluene; 3,5-dimethylnitrosobenzene; o-nitrosoethylbenzene; o-nitrosostyrene; o-nitrosoanisole; m-nitrosoanisole; p-nitrosoanisole; o-nitrosophenetol; m-nitrosophenetol; p-nitrosophenetol; o-fluoronitrosobenzene; m-fluoronitrosobenzene; p-fluoronitrosobenzene; o-chrolonitrosobenzene; m-chrolonitrosobenzene; p-chloronitrosobenzene; o-bromonitrosobenzene; m-bromonitrosobenzene; p-bromonitrosobenzene; and the like can be exemplified.

A heterocyclic compound used in the process for producing α-aminooxyketone compounds of the present invention is shown in the general formula (I) wherein X1, X2 and X3 independently represent nitrogen, carbon, oxygen or sulfur; Z represents a substituted or unsubstituted 5- to 10-membered ring, and is a compound in which a 5-membered heterocycle and 5- to 10-membered heterocycle both having nitrogen atom at the respective α position are bound through carbons constituting the heterocycles. The heterocyclic compound is also called a N-H acid-N-H base combined catalyst where the NH functional group at the α position of the heterocycle acts as an acid and the NH functional group at the α position of the cycloalkyl heterocycle acts as a base. As a 5-membered heterocycle (acid ring) of the heterocyclic compound, tetrazole, 1,2,3-triazole, 1,2,4-triazole, pyrazole, pyrazoline, imidazole, indazoline, thiotriazoline and oxatriazoline can be exemplified, and 1H-tetrazole as shown in the genenral formula (II) is especially preferable as a heterocyclic compound. As a 5-10 membered heterocycle (base ring), pyrrolidine, piperidine, hexamethyleneimine, heptamethyleneimine, oxazoline and oxazole can be exemplified; and as a substituent of these heterocycles, alkyl groups such as methyl, ethyl, n-propyl, isopropyl, n-butyl, isobutyl, sec-butyl, and t-butyl, alkoxy groups such as methoxy and ethoxy, phenyl groups, aryl groups such as condensed ring system condensed to heterocycle can be exemplified. Smaller substituents are preferable since a compound with a bulky heterocyclic moiety including substituents would lower the yield.

As the above heterocyclic compound, specifically 5-(2'-pyrrolidinyl)-1H-1,2,3,4-tetrazole; 5-(4H,5H-2'-oxazolyl)-1H-1,2,3,4-tetrazole;
5-(2'-piperidinyl)-1H-1,2,3,4-tetrazole;
5-(benzo[c]-2'-piperidinyl)-1H-1,2,3,4-tetrazole;
5-2'-pyrolidinyl-1H-1,2,3-triazole;
5-2'-pyrolidinyl-1H-1,2,4-triazole;
2-2'-pyrolidinyl-1H-imidazole;
5-2'-pyrolidinyl-1H-imidazole;
5-2'-pyrolidinyl-1H,4H,5H-1,2,3,4-thiotriazoline;
5-2'-pyrolidinyl-4H,5H-1,2,3,4-oxatriazoline; and 5-2'-pyrolidinyl-4H,5H-pyrazoline; can be exemplified. Particularly, 5-(2'-pyrolidinyl)-1H-1,2,3,4-tetrazole as shown in the formura (III) can be preferably exemplified.

The heterocyclic compound can be prepared from natural or synthesized proline. The tetrazole derivative shown in the formula (III) can be prepared by a previous method (Roczniki Chemii Ann. Soc. Chim. Polpnorum, 1971, 45, 967; J. Med. Chm. 1985, 28, 1067). Thus, N-(benzyloxycarbonyl)-L-proline, which is commercially available as a carboxylic acid of proline having nitrogen atom protected by a benzyloxycarbonyl group, is converted to an amide via a reaction with ammonia and dehydrated with phosphoryl chloride to give nitrile. The obtained nitrile is reacted with sodium azide to give a tetrazole, and finally the N-benzyloxycarbonyl group is deprotected with HBr/AcOH or Pd/C, H2 to give a tetrazole derivative shown in the formula (III). The tetrazole derivative can also be obtained according to the previous method from Organic Letters, 2001, Vol.3, No.25, 4091-4094; Organic Letters, 2002, Vol. 4 No. 15, 2525- 2527; and the like.

A process for producing α-aminooxyketone compounds of the present invention is a method of performing the reaction of carbonyl compound and nitroso compound generally in solvent in the presence of the heterocyclic compound shown in the general formula (I) or preferably in the presence of the tetrazole derivative shown in the formula (III). The amount of nitroso compound can be in a range of 2-4 mol equivalents, preferably 2.5-3.5 mol equivalents for the carbonyl compound, and the amount of catalyst containing the heterocyclic compound shown in the formula (I) can be 1-30 mol%, preferably 2-20 mol% for carbonyl substrate. As a solvent, water; halogenated hydrocarbons such as dichloromethane, chloroform, dichloroethane, and chlorobenzene; aromatic hydrocarbons such as benzene, toluene, xylene; aliphatic hydrocarbons such as cyclohexane, n-hexane, and n-heptane; esters such as ethylacetate ; nitriles such as acetonitrile; dimethylsulfoxide; and the like can be exemplified; and among them dimethylsulfoxide and acetonitrile are preferable. The amount of solvent can be 15-30 equivalents but the reaction can be done without solvent.

The reaction temperature can be 0-50° C, preferably 20-30°C and the reaction can be done at room temperature. The reaction time can be 30 minutes to 3 hours, for example, around 1 hour, and a method stirring in the open air can be exemplified. Strict conditions are not required for a reaction, furthermore, water does not suppress the reaction to proceed, so there is no need for dehydrating the material and catalyst, and the reaction is easy to be controlled. After the reaction is complete, the resulting product was extracted with ethylacetate and the like, and dried and purified by known methods.

In a process for producing α-aminooxyketone compounds of the present invention, carbonyl compounds such as methylisopropylketone and acetophenone, and aldehydes such as water-soluble aldehyde and aldehyde that forms hydrate easily, which could not be used for the materials in conventional methods, can be used as materials. Since there are not many limitations for carbonyl compounds used as a material, the resulting products, α-aminooxyketone compounds, range over a wide scope and have a high chemical yield and optical purity. As an absolute configuration of asymmetric carbons of the resulting product, α-aminooxyketone compound; where aldehyde is used as a material, the resulting product has a R-configuration; where ketone is used as a material, the resulting product has a S-configuration.

Specific examples of the α-aminooxyalketone obtained from the present invention are as follows:
(N-isobutylaminooxy)acetaldehyde;
[N-(1,1-dimethylbutyl)]aminooxyacetaldehyde;
(N-phenylaminooxy)acetaldehyde;
2-(N-isobutylaminooxy)propanal;
2-[N-(1,1-dimethylbutyl)aminooxy]propanal;
2-N-phenylaminooxypropanal; 2-(N-isobutylaminooxy)butanal;
2-[N-(1,1-dimethylbuthyl)aminooxy]butanal;
2-(N-phenylaminooxy)butanal;
2-(N-isobutylaminooxy)-2-methylpropanal;
2-[N-(1,1-dimethylbuthyl)aminooxy]-2-methylpropanal;
2-(N-phenylaminooxy)-2-methylpropanal;
2-(N-isobutylaminooxy)-4-methylbutanal;
2-[N-(1,1-dimethylbutyl)aminooxy]-4-methylbutanal;
2-(N-phenylaminooxy)-4-methylbutanal;
2-(N-isobutylaminooxy)hexanal;
2-[N-(1,1-dimethylbutyl)aminooxy]hexanal;
2-(N-phenylaminooxy)hexanal;
2-(N-isobutylaminooxy)heptanal;
2-[N-(1,1-dimethylbutyl)aminooxy]heptanal;
2-(N-phenylaminooxy)heptanal;
2-(N-isobutylaminooxy)octanal;
2-[N-(1,1-dimethylbutyl)aminooxy]octanal;
2-(N-phenylaminooxy)octanal; 2-(N-isobutylaminooxy)nonanal;
2-[N-(1,1-dimethylbutyl)aminooxy]nonanal;
2-(N-phenylaminooxy)nonanal; 2-(N-isobutylaminooxy)decanal; 2-[N-(1,1-dimethylbutyl)aminooxy]decanal;
2-(N-phenylaminooxy)decanal;
2-(N-isobutylaminooxy)undecanal;
2-[N-(1,1-dimethylbutyl)aminooxy]undecanal;
2-(N-phenylaminooxy)propanal;
2-(N-isobutylaminooxy)dodecanal;
2-[N-(1,1-dimethylbutyl)aminooxy]dodecanal;
2-(N-phenylaminooxy)dodecanal;
2-(N-isobutylaminooxy)tridecanal; 2-[N-(1,1-dimethylbutyl)aminooxy]tridecanal; and 2-(N-phenylaminooxy)tridecanal.

Further specific examples of the α-aminooxyketone compound obtained from the present invention are as follows:
2,3-bis(N-isobutylaminooxy)butanedial;
2,3-bis[N-(1,1-dimethylbutyl)aminooxy]butanedial;
2,3-bis[N-phenylaminooxy]butanedial;
2-N-isobutylaminooxy-2-propenal;
2-N-(1,1-dimethylbutyl)aminooxy-2-propenal;
2-N-phenylaminooxy-2-propenal;
2-N-isobutylaminooxy-2-butenal;
2-N-(1,1-dimethylbutyl)aminooxy-2-butenal;
2-N-phenylaminooxy-2-butenal;
3-phenyl-2-N-isobutylaminooxy-2-propenal;
3-phenyl-2-N-(1,1-dimethylbutyl)aminooxy-2-propenal; and
3-phenyl-2-N-phenylaminooxy-2-propenal.

Furthermore, examples of the α-aminooxyketone obtained from the present invention are as follows:
(N-isobutylaminooxy)acetone;
[N-(1,1-dimethylbutyl)aminooxy]acetone;
(N-phenylaminooxy)acetone;
3-(N-isobutylaminooxy)butane-2-one;
3-[N-(1,1-dimethylbutyl)aminooxy]butane-2-one;
3-(N-phenylaminooxy)butane-2-one;
3-(N-isobutylaminooxy)pentane-2-one;
3-[N-(1,1-dimethylbutyl)aminooxy]pentane-2-one;
3-(N-phenylaminooxy)pentane-2-one;
3-(N-isobutylaminooxy)-4-methylbutane-2-one;
3-[N-(1,1-dimethylbutyl)aminooxy]-4-methylbutane-2-one;
3-(N-phenylaminooxy)-4-methylbutane-2-one;
3-(N-isobutylaminooxy)hexane-2-one;
3-[N-(1,1-dimethylbutyl)aminooxy]hexane-2-one;
3-(N-phenylaminooxy)hexane-2-one;
3-(N-isobutylaminooxy)-4-methylpentane-2-one;
3-[N-(1,1-dimethylbutyl)aminooxy]-4-methylpentane-2-one;
3-(N-phenylaminooxy)-4-methylpentane-2-one;
2-(N-isobutylaminooxy)pentane-3-one;
2-[N-(1,1-dimethylbutyl)aminooxy]pentane-3-one;
2-(N-phenylaminooxy)pentane-3-one;
2-(N-isobutylaminooxy)-2,4-dimethylpentane-3-one;
2-[N-(1,1-dimethylbutyl)aminooxy]-2,4-dimethylpentane-3-one;
2-(N-phenylaminooxy)-2,4-dimethylpentane-3-one;
3-(N-isobutylaminooxy)undecane-2-one;
3-[N-(1,1-dimethylbutyl)aminooxy]undecane-2-one; and
3-(N-phenylaminooxy)undecane-2-one.

Further examples of the α-aminooxyketone compound obtained from the present invention are as follows:
3-N-isobutylaminooxy-3-butene-2-one;
3-N-(1,1-dimethylbutyl)aminooxy-3-butene-2-one;
3-N-phenylaminooxy-3-butene-2-one;
3-N-isobutylaminooxy-4-methyl-3-pentene-2-one;
3-N-(1,1-dimethylbutyl)aminooxy-4-methyl-3-pentene-2-one;
3-N-phenylaminooxy-4-methyl-3-pentene-2-one;
1-fluoro-1-(N-isobutylaminooxy)acetone;
1-fluoro-1-[N-(1,1-dimethylbutyl)aminooxy]acetone;
1-fluoro-1-(N-phenylaminooxy)acetone;
1-chloro-1-(N-isobutylaminooxy)acetone;
1-chloro-1-[N-(1,1-dimethylbutyl)aminooxy]acetone;
1-chloro-1-(N-phenylaminooxy)acetone;
3-(N-isobutylaminooxy)-2,4-pentanedione;
3-[N-(1,1-dimethylbutyl)aminooxy]-2,4-pentanedione;
3-(N-phenylaminooxy)-2,4-pentanedione;
2-(N-isobutylaminooxy)cyclobutanone;
2-[N-(1,1-dimethylbutyl)aminooxy]cyclobutanone;
2-(N-phenylaminooxy)cyclobutanone;
2-(N-isobutylaminooxy)cyclopentanone;
2-[N-(1,1-dimethylbutyl)aminooxy]cyclopentanone;
2-(N-phenylaminooxy)cyclopentanone;
2-(N-isobutylaminooxy)cyclohexanone;
2-[N-(1,1-dimethylbutyl)aminooxy]cyclohexanone;
2-(N-phenylaminooxy)cyclohexanone;
2-(N-isobutylaminooxy)-2-methylcyclohexanone;
2-[N-(1,1-dimethylbutyl)aminooxy]-2-methylcyclohexanone;
2-(N-phenylaminooxy)-2-methylcyclohexanone;
2-(N-isobutylaminooxy)cyclodecanone;
2-[N-(1,1-dimethylbutyl)aminooxy]cyclodecanone;
2-(N-phenylaminooxy)cyclodecanone;
1-(N-isobutylaminooxy)-2-norbornanone;
1-[N-(1,1-dimethylbutyl)aminooxy]-2-norbornanone;
1-(N-phenylaminooxy)-2-norbornanone;
1-(N-isobutylaminooxy)-2-adamantanone;
1-[N-(1,1-dimethylbutyl)aminooxy]-2-adamantanone; and
1-(N-phenylaminooxy)-2-adamantanone.

And more examples are as follows:
2-(N-isobutylaminooxy)-4-tetrahydropyranone;
2-[N-(1,1-dimethylbutyl)aminooxy]-4-tetrahydropyranone;
2-(N-phenylaminooxy)-4-tetrahydropyranone;
7-(N-isobutylaminooxy)-spiro[4.5]-1,4-dioxy-decane-8-one;
7-[N-(1,1-dimethylbutyl)aminooxy]-spiro[4.5]-1,4-dioxy-deca ne-8-one;
7-(N-phenylaminooxy)-spiro[4.5]-1,4-dioxy-decane-8-one;
3-(N-isobutylaminooxy)-1-benzylcarbonylpiperidine-4-one;
3-[N-(1,1-dimethylbutyl)aminooxy]-1-benzylcarbonylpiperidin e-4-one;
3-(N-phenylaminooxy)-1-benzylcarbonylpiperidine-4-one;
3-(N-isobutylaminooxy)-4-phenylbutane-2-one;
3-[N-(1,1-dimethylbutyl)aminooxy-4-phenylbutane-2-one;
3-(N-phenylaminooxy)-4-phenylbutane-2-one;
2-(N-isobutylaminooxy)-1-indanone;
2-[N-(1,1-dimethylbutyl)aminooxy]-1-indanone;
2-(N-phenylaminooxy)-1-indanone:
1-(N-isobutylaminooxy)-2-indanone;
1-[N-(1,1-dimethylbutyl)aminooxy-2-indanone;
1-(N-phenylaminooxy)-2-indanone;
2-(N-isobutylaminooxy)-1-ketotetrahydronaphthalene;
2-[N-(1,1-dimethylbutyl)aminooxy-1-ketotetrahydronaphthalen e; 2-(N-phenylaminooxy)-1-ketotetrahydronaphthalene;
1-(N-isobutylaminooxy)-2-ketotetrahydronaphthalene;
1-[N-(1,1-dimethylbutyl)aminooxy]-2-ketotetrahydronaphthale ne; 1-(N-phenylaminooxy)-2-ketotetrahydronaphthalene;
1-(N-isobutylaminooxy)-7-methoxy-2-ketotetrahydronaphthalen e;
1-[N-(1,1-dimethylbutyl)aminooxy-7-methoxy-2-ketotetrahydro naphthalene;
1-(N-phenylaminooxy)-7-methoxy-2-ketotetrahydronaphthalene;
2'-(N-isobutylaminooxy)-1'-acetophenone;
2'-[N-(1,1-dimethylbutyl)aminooxy]-1'-acetophenone;
2'-(N-phenylaminooxy)-1'-acetophenone;
2'-(N-isobutylaminooxy)-1'-propiophenone;
2'-[N-(1,1-dimethylbutyl)aminooxy]-1'-propiophenone;
2'-(N-phenylaminooxy)-1'-propiophenone;
2-(N-isobutylaminooxy)-1,2-bisphenylethane-1-one;
2-[N-(1,1-dimethylbutyl)aminooxy]-1,2-bisphenylethane-1-one;
2-(N-phenylaminooxy)-1,2-bisphenylethane-1-one;
1-(N-isobutylaminooxy)-1,3-bisphenylpropane-2-one;
1-[N-(1,1-dimethylbutyl)aminooxy]-1,3-bisphenylpropane-2-on e; 1-(N-phenylaminooxy)-1,3-bisphenylpropane-2-one;
6-(N-isobutylaminooxy)-3,4-dimethylacetophenone;
6-[N-(1,1-dimethylbutyl)aminooxy]-3,4-dimethylacetophenone;
6-(N-phenylaminooxy)-3,4-dimethylacetophenone;
3'-(N-isobutylaminooxy)-2'-acetonaphthone,
3'-[N-(1,1-dimethylbutyl)aminooxy]-2'-acetonaphthone;
3'-(N-phenylaminooxy)-2'-acetonaphthone;
3'-(N-isobutylaminooxy)-2'-chloroacetonaphthone;
3'-[N-(1,1-dimethylbutyl)aminooxy]-2'-acetonaphtone; and
3'-(N-phenylaminooxy)-2'-acetonaphtone.

Further, the process for producing α-hydroxyketone compounds of the present invention is a process using an α-aminooxyketone compound obtained from the above mentioned process for producing α-aminooxyketone compounds of the present invention in a solvent with the use of CuSO₄ as a catalyst, and to which known methods can be applied to convert aminooxyketone compound to hydroxyketone compound. Alcohols like methanol and ethanol can be exemplified as a solvent to use. The reaction temperature can be about O-25 °C, and the reaction time can be about 3-10 hours.

The present invention will be explained in detail in the following by referring to the examples, but the technical scope of the present invention will not be limited to these.

### Example 1

Pyrrolidine-2-tetrazole 1 was prepared from proline. One mL dimethyl sulfoxide (DMSO) solution at room temperature (25-30 °C) in which 5 mol% Pyrrolidine-2-tetrazole and 1.5 mmol (3 equivalents) cyclohexanone was dissolved was added dropwise 1 mL DMSO solution with 0.5 mmol (1 equivalent) nitrosobenzene for 1 hour. The mixture was stirred at room temperature and allowed to react for 1 hour. The nitrosobenzene was completely consumed, as determined by TLC (hexane/ethyl acetate = 3/1). The desired product (4), 2-(N-phenylaminooxy)-1-cyclohexanone was obtained. It was 94% chemical yield and >99%ee optical purity. The results are shown in Table 1. In Table 1, chemical yield ratio (4/6) shows the yield ratio of isolated isomer, optical purity (ee%) of the desired product shows the measurement of HPLC, and absolute configuration (R/S) of asymmetric carbon of the desired products shows the yield of diol converted from the desired products. Even where the amount of catalyst 1 to be used was changed to 3 mol% and 2 mol% , the reactions progressed. The chemical yield of the desired products was 72% and 50% respectively, but the optical purities were >99%ee.

### (comparative example 1)

Reaction was performed in the same manner as Example 1 with the exception of using proline as a catalyst. The chemical yield of the resulting desired product stayed at 35%, and the production of double additional product (5a) of about 2% of 2,6-bis(N-phenylaminooxy)-1-cyclohexanone was identified.

### Example 2

Reaction was performed in the same manner as Example 1 with the exception of using tetrahydropyrane-4-one as a carbonyl compound. Chemical yield and optical purity of the desired products are shown in Table 1.

### Example 3

Reaction was performed in the same manner as Example 1 with the exception of using spiro[4.5]-1,4-dioxy-decane-8-one as a carbonyl compound. Chemical yield and optical purity of the desired products are shown in Table 1.

### Example 4

Reaction was performed in the same manner as Example 1 with the exception of using 1-benzylcarbonylpiperidine-4-one as a carbonyl compound. Chemical yield and optical purity of the desired products are shown in Table 1.

### Example 5

Reaction was performed in the same manner as Example 1 with the exception of using methylethylketone as a carbonyl compound and the amount of 20 mol% catalyst. Chemical yield and optical purity of the desired products are shown in Table 1.

### Example 6

Reaction was performed in the same manner as Example 1 with the exception of using phenylpropionaldehyde as a carbonyl compound, acetonitrile as a solvent, and the amount of 10 mol% catalyst. Chemical yield and optical purity of the desired products are shown in Table 1. In the Table, chemical yield of the desired products shows the yield of primary alcohol obtained by reduction of the desired products.

### Example 7

Reaction was performed in the same manner as Example 6 with the exception of using isobutyraldehyde as a carbonyl compound and the amount of 20 mol% catalyst. Chemical yield and optical purity of the desired products are shown in Table 1.

### Example 8

Reaction was performed in the same manner as Example 6 with the exception of using caproicaldehyde as a carbonyl compound. Chemical yield and optical purity of the desired products are shown in Table 1.

**[Table 1]**

| | | | | | | | |
|---|---|---|---|---|---|---|---|
| 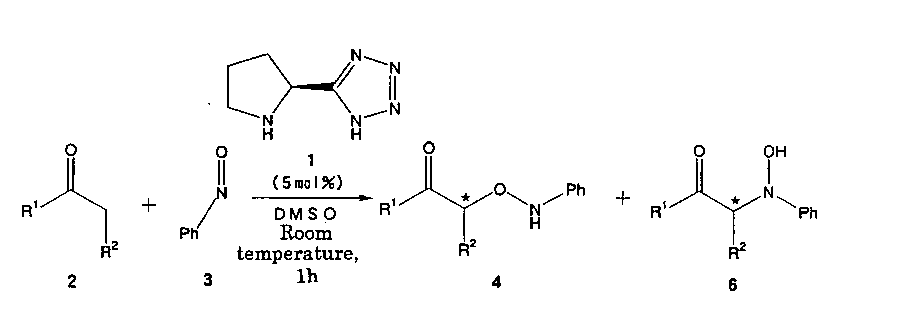 | | | | | | | |

| Example | 2 | 1 (mol%) | Solvent | Yield (%) | Yield ratio4/6 | Optical purity4 (ee%) | R/Sconfiguration |
|---|---|---|---|---|---|---|---|
| 1 | 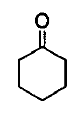 | 5 | DMSO | 94 | >99 / - | >99 | S |
| 2 | 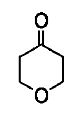 | 5 | DMSO | 87 | >99 / - | >99 | S |
| 3 | 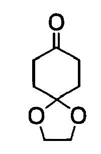 | 5 | DMSO | 97 | >99/- | 99 | S |
| 4 | 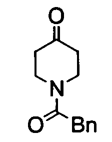 | 5 | DMSO | 95 | >99/- | >99 | S |
| 5 | 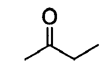 | 20 | DMSO | 75 | 72 / 28 | >99 | S |
| 6 | 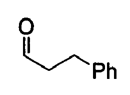 | 10 | MeCN | 67 | >99 / - | 98 | R |
| 7 | 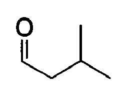 | 20 | MeCN | 65 | >99 / - | 98 | R |
| 8 | 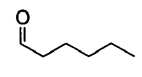 | 10 | MeCN | 69 | >99 / - | 98 | R |

### Example 9

DMSO solution containing 1 mmol of the resulting products from Example 1 was directly cooled to 0°C, added 47.9 mg (0.3 mmol) CuSO₄ and 3.0 mL methanol, and stirred at 0°C for 10 hours. The reaction mixture was added 20 mL cooled saline solution as aftertreatment, and extracted 3 times with 10 mL ethyl acetate. The extracted organic phase was all collected and washed with saline solution, filtered after drying with Na₂SO₄, and distilled the resulting filtrate with evaporator under reduced pressure. After the residue was purified with silica gel chromatography (developing solvent: ethyl acetate/hexane), corresponding α-hydroxyketone was obtained. The yield was 90 %, and optical yield was 99 % or more.

## Claims

1. A process for producing an α-aminooxyketone compound wherein a carbonyl compound is reacted with a nitroso compound by using a catalyst containing a heterocyclic compound shown in the general formula (I) (wherein: X1, X2 and X3 independently represent nitrogen, carbon, oxygen or sulfur; Z represents a substituted or unsubstituted 5- to 10-membered ring).

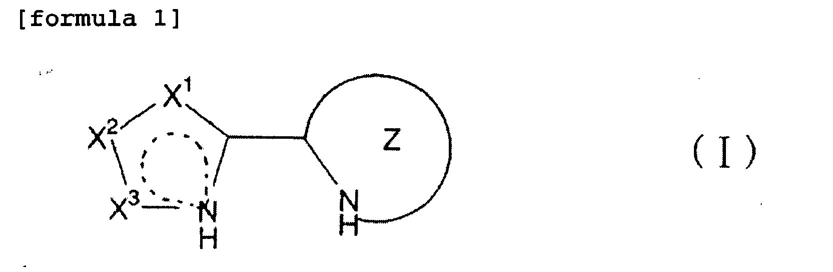

2. The process for producing an α-aminooxyketone compound according to claim 1, wherein the heterocyclic compound is a tetrazole derivative shown in the general formula (II) (wherein Z represents a substituted or unsubstituted 5- to 10-membered ring).

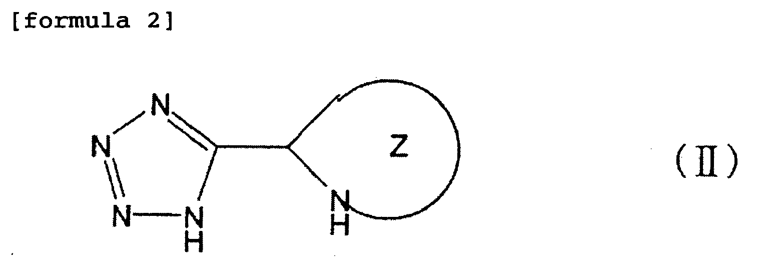

3. The process for producing an α-aminooxyketone compound according to claim 2, wherein the tetrazole derivative is a compound shown in the general formula (III).

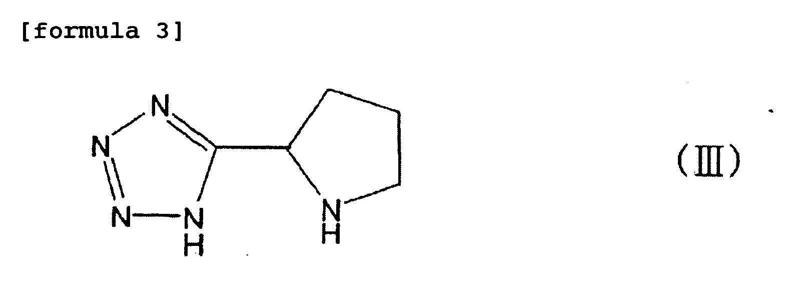

4. The process for producing an α-aminooxyketone compound according to any one of claims 1-3, wherein the nitroso compound is nitrosobenzene.

5. The process for producing an α-aminooxyketone compound according to any one of claims 1-4, wherein the carbonyl compound is a compound shown in the general formula (IV) (wherein R1 and R2 independently represent hydrogen, or a substituted or unsubstituted alkyl group, a substituted or unsubstituted alkenyl group, a substituted or unsubstituted alkoxy group, a substituted or unsubstituted amide group, or a substituted or unsubstituted aryl group; and R1 and R2 may bind to each other to form a ring).

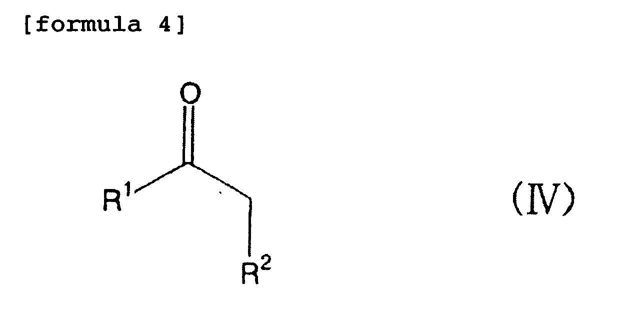

6. The process for producing an α-aminooxyketone compound according to claims 5, wherein the α-aminooxyketone compound contains R asymmetric carbon, where R1 represents hydrogen in the general formula (IV).

7. The process for producing an α-aminooxyketone compound according to claims 5, wherein the α-aminooxyketone compound contains S asymmetric carbon; where R1 represents a substituted or unsubstituted alkyl group, a substituted or unsubstituted alkenyl group, a substituted or unsubstituted alkoxy group, a substituted or unsubstituted amide group, or a substituted or unsubstituted aryl group, and either one of these groups bind to R2 to form a ring in the general formula (IV).

8. The process for producing an α-aminooxyketone compound according to any one of claims 1-7, wherein a solvent containing dimethyl sulfoxide or methyl nitrile, or both of them is used.

9. The process for producing an α-aminooxyketone compound according to any one of claims 1-8, wherein reaction is performed at room temperature (20-30 °C).

10. A process for producing an α-hydroxyketone compound wherein an α-aminooxyketone compound obtained from the process according to any one of claims 1-9 is reacted in a solvent by using CuSO₄ as a catalyst.

11. A catalyst for producing an α-aminooxyketone compound by which a carbonyl compound is reacted with a nitroso compound, wherein the catalyst contains a heterocyclic compound shown in the general formula (I) (wherein: X1, X2 and X3 independently represent nitrogen, carbon, oxygen or sulfur; Z represents a substituted or unsubstituted 5- to 10-membered ring).

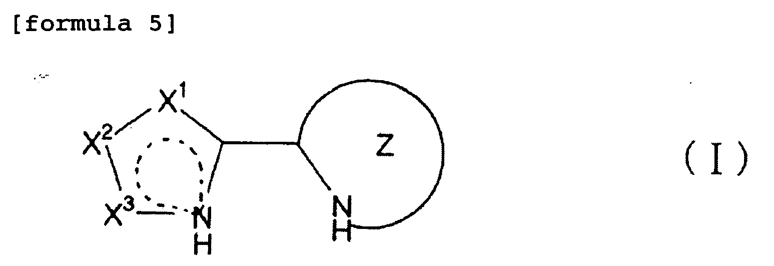

12. The catalyst for producing an α-aminooxyketone compound according to claim 11, wherein the heterocyclic compound is a tetrazole derivative shown in the general formula (II) (wherein Z represents a substituted or unsubstituted 5- to 10-membered ring).

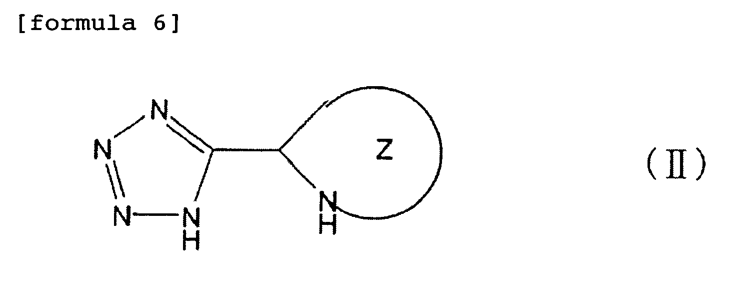

13. The catalyst for producing an α-aminooxyketone compound according to claim 12, wherein the tetrazole derivative is a compound shown in the general formula (III).

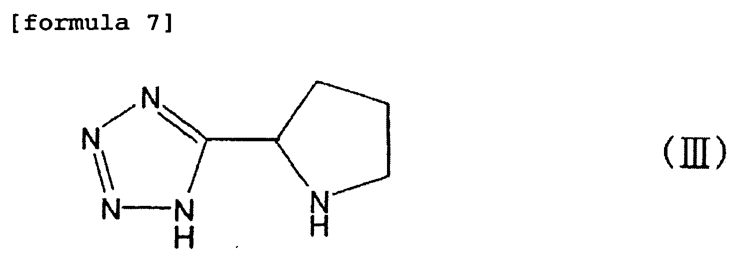

14. The catalyst for producing an α-aminooxyketone compound according to any one of claims 11-13, wherein the nitroso compound is nitrosobenzene.

15. The catalyst for producing an α-aminooxyketone compound according to any one of claims 11-14, wherein the carbonyl compound is a compound shown in the general formula (IV) (wherein R1 and R2 independently represent hydrogen, or a substituted or unsubstituted alkyl group, a substituted or unsubstituted alkenyl group, a substituted or unsubstituted alkoxy group, a substituted or unsubstituted amide group, or a substituted or unsubstituted aryl group; and R1 and R2 may bind to each other to form a ring).

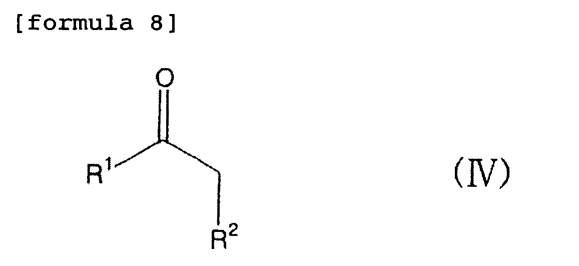

16. The catalyst for producing an α-aminooxyketone compound according to claim 15, wherein the α-aminooxyketone compound contains R asymmetric carbon, where R1 represents hydrogen in the general formula (IV).

17. The catalyst for producing an α-aminooxyketone compound according to claim 15, wherein the α-aminooxyketone compound contains S asymmetric carbon, where R1 represents a substituted or unsubstituted alkyl group, a substituted or unsubstituted alkenyl group, a substituted or unsubstituted alkoxy group, a substituted or unsubstituted amide group, or a substituted or unsubstituted aryl group, or where either one of these groups bind to R2 to form a ring in the general formula (IV).
